# EUROPEAN PATENT APPLICATION

(11) **EP 4 183 869 A1**
(43) Date of publication of application: **24.05.2023**
(21) Application number: 21841342.5
(22) Date of filing: 14.01.2021
(51) Int. Cl.: C12N 5/077, A61F 2/06, A61F 2/12, A61K 35/35, A61P 9/00

(54) **METHOD FOR PRODUCING MATURE ADIPOCYTE-CONTAINING COMPOSITION**

(30) Priority: 17.07.2020 JP 2020122512
(71) Applicant: Medical Corporation Yanaga Clinic, Fukuoka-shi, Fukuoka 810-0001 (JP)
(72) Inventor: YANAGA, Hiroko, Fukuoka-shi, Fukuoka 810-0001 (JP); YANAGA, Katsu, Fukuoka-shi, Fukuoka 810-0001 (JP)
(74) Representative: van Dam, Vincent
(86) International application number: PCT/JP2021/001133
(87) International publication number: WO 2022/014071

(57) **Abstract**

[Problem] To provide a mature adipocyte-containing composition that has excellent revascularization ability and is less likely to cause inflammation.

[Solution] This method for producing a mature adipocyte-containing composition comprises: a cleavage-filtration step for cleaving and filtering fatty tissue that is not treated with an enzyme and obtaining a minimal fat; a minimal fat culture step for culturing the minimal fat, after the cleavage-filtration step, by means of a culture medium, and obtaining mature adipocytes; and a mature adipocyte-containing composition obtaining step for obtaining a mature adipocyte-containing composition including the mature adipocytes and a conditioned medium obtained from the minimal fat culture step, wherein the culture medium contains an autoserum or contains FBS, hydrocortisone, and FGF-2.

## Description

### Technical Field

This invention relates to a method for producing a mature adipocyte-containing composition. More specifically, this invention relates to a method for producing a mature adipocyte-containing composition including a mature adipocyte and a conditioned medium (e.g., a supernatant of the mature adipocyte), in which a fat graft survival rate is increased by improving revascularization of an adipose tissue for grafting.

### Background Art

Fat grafting that is performed after removing breast cancer as breast reconstruction surgery is considered safe without promoting cancer metastasis. Thus, the breast reconstruction surgery is commonly performed in plastic and cosmetic surgical fields. However, due to a large quantity of breast tissues, a graft survival rate of adipose tissue pieces obtained by simply suctioning adipose tissues from the living body is only 30 to 45% (Non Patent Literature 1 by Zhou et al.).

The low survival rate of the suctioned adipose tissue pieces in the recipient environment is mainly caused by a lack of feeding blood vessels. The grafted adipose tissue pieces, which have been separated from the original feeding blood vessels, are undernourished and undergo necrosis. It is well known that the adipose tissue pieces are recognized as a foreign object after grafting, followed by absorption, cyst formation, and calcification. Further, it has been reported that, as a method for improving the survival rate, a method in which the adipose tissues are grafted together with adipose-derived stem cells (ASCs) present in a small vascular fraction (SVF) included in a stromal vascular fraction at the perivascular region of the adipose tissues (cell-assisted lipotransfer: CAL) (Non Patent Literature 2 by Eto et al.). In this method, the SVF adipose-derived progenitor stem cells present at the perivascular region are obtained by treating the suctioned adipose tissues with an enzyme and removing fat components and then grafted together with the suctioned adipose tissues. The SVF is characterized by having high self-renewal capacity and multilineage potential (Non Patent Literature 3 by Zuk PA et al.).

Zhou et al. have performed systematic review on 17 articles involving 387 cases (Non Patent Literature 1 by Zhou et al.). The fat survival rate was significantly higher in the CAL group than the group in which the suctioned fats alone were grafted (60% vs. 45%, p=0.0096). The CAL significantly increased the facial fat survival rate (by 19%) and decreased the incidence of multiple operations (by 13.6%). However, in the breast fat grafting, the fat survival rate increased only by 9% with no statistical significance.

On the other hand, the CAL (cell-assisted lipotransfer) in breast cases (Non Patent Literature 2 by Eto et al.) was associated with the higher incidence of complications as compared to facial cases (p<0.001). The CAL is a method in which, by utilizing the adipose-derived stem cells (ASCs) present in the stromal vascular fraction (SVF: stromal vascular fraction) obtained by treating the adipose tissues with an enzyme, the ASCs that cause revascularization and the adipose tissues are grafted together. However, the CAL often fails due to absorption of the grafted fats. The reason for this can be found in that the grafted adipose tissues are separated from the original feeding blood vessels and many of the adipose tissues become undernourished and undergo necrosis although some of the adipose tissues (the fats within 300 µm) survive.

Thus far, two types of cells, namely, the adipose-derived stem cells (ASCs) as the adipose progenitor cells and the dedifferentiated fat (DFAT) cells, have been successfully developed as cells for revascularization.

The ASCs are the adipose progenitor cells, but they are also multipotent stem cells that differentiate into not only adipocytes, but also osteocytes, myocytes, chondrocytes, and the like (Non Patent Literature 3 by Zuk PA et al.). Further, it has been reported that the ASCs promote cancer metastasis, thus the ASCs are not suitable for the fat grafting commonly performed in the breast reconstruction surgery after removing breast cancer. Further, the ASCs induce a large quantity of TNF-α under low oxygen conditions and thus may cause inflammation, making it difficult to prevent the absorption after grafting caused by induction of the inflammation. As a result, the ASCs are not suitable for grafting.

Further, a factor through which the DFAT cells induce vascularization has not been identified. A lack of a clear mechanism of this process also hinders the application to humans.

### Citation List

### Non Patent Literature

Non Patent Literature 1: Zhou Y, Wang J, Li H, et al. Efficacy and Safety of Cell-Assisted Lipotransfer: A Systematic Review and Meta-Analysis. Plast Reconstr Surg 2016: 137: 44e-57e.
Non Patent Literature 2: Eto H, et al.: The fate of adipocyte after non vascularized fat grafting: Evidence of early death and replacement of adipocytes. Plast Reconstr Surg. 2012; 129(5): 1081-92.
Non Patent Literature 3: Zuk PA. et al.: Human adipose tissue is a source of multipotent stem cells. Mol Biol Cell 2001; 13: 4279-95.

### Summary of Invention

### Technical Problem

In view of the above-described circumstances, a mature adipocyte-containing composition that has high revascularization ability has been desired. Further, a mature adipocyte-containing composition that is less likely to induce inflammation has been desired.

Further, a revascularization promoting agent, a composition used as a substitute for subcutaneous adipose tissues, and a fat-containing agent used in augmentation mammaplasty, breast reconstruction surgery, or tissue recess reconstruction surgery, including such a mature adipocyte-containing composition have been desired. Solution to Problem

The present invention is essentially based on findings in Examples that a composition including mature adipocytes, which are obtained by shredding and filtering adipose tissues and culturing microfats using a medium, and a conditioned medium (a culture supernatant), which is obtained during the above culturing, contains a large quantity of chemokines/cytokines that promote vascularization, such as IL-8, GRO, MCP-1, or VEGF (vascular endothelial growth factor) and that, when this composition is used, breast reconstruction surgery, for example, is extremely successful.

A first aspect of the invention described in this specification is related to a method for producing a mature adipocyte-containing composition. This method includes a shredding-filtration step, a microfat culture step, and a mature adipocyte-containing composition obtaining step.

The shredding-filtration step is a step of obtaining microfats by shredding and filtering adipose tissues which are not treated with an enzyme.

The microfat culture step is a step of obtaining mature adipocytes by culturing the microfats using a culture medium after the shredding-filtration step. The culture medium includes hydrocortisone and FGF-2.

The mature adipocyte-containing composition obtaining step is a step of obtaining a mature adipocyte-containing composition including the mature adipocytes and a conditioned medium obtained through the microfat culture step.

In a preferable example, the adipose tissues are prepared by washing suctioned fats and then removing blood components.

In a preferable example, the shredding-filtration step includes a step of removing a precipitated stromal vascular fraction (SVF) by performing centrifugal separation.

In a preferable example, the mature adipocytes express each of IL-8, GRO, and MCP-1, a family of chemokines/cytokines, 4 times or more (preferably 10 times or more, more preferably 20 times or more) higher than IL-6. The conditioned medium preferably contains these chemokines/cytokines. The conditioned medium preferably contains vascular endothelial growth factor (VEGF) 0.5 times or more to 1.5 times or less higher than IL-6.

In a preferable example, the mature adipocyte-containing composition is used as a substitute for subcutaneous adipose tissues. Further, the mature adipocyte-containing composition may be used in augmentation mammaplasty, breast reconstruction surgery, or tissue recess reconstruction surgery.

A second aspect of the invention described in this specification is related to a composition used as a substitute for subcutaneous adipose tissues. This composition is the mature adipocyte-containing composition produced by any of the above methods for producing the mature adipocyte-containing composition and used as a substitute for subcutaneous adipose tissues. This composition is used in, for example, augmentation mammaplasty, breast reconstruction surgery, or tissue recess reconstruction surgery.

A third aspect of the invention described in this specification is related to a revascularization promoting agent. This revascularization promoting agent includes the mature adipocyte-containing composition obtained by, for example, any of the above methods for producing the mature adipocyte-containing composition. The mature adipocyte-containing composition includes vascular endothelial growth factor receptor (VEGFR) 2 or a positive cell thereof, and platelet-derived growth factor receptor (PDGFR) β.

### Advantageous Effects of Invention

The mature adipocyte-containing composition obtained in the first aspect of the invention normally exhibits high expression of IL-8, GRO, MCP-1, or VEGF having high vascularization promoting ability. Thus, this mature adipocyte-containing composition has high revascularization ability.

Further, as described above, the ASCs induce a large quantity of TNF-α under low oxygen conditions and thus may induce inflammation. Therefore, the ASCs are not suitable for grafting. While the mature adipocyte-containing composition obtained in the first aspect of the invention exhibits high expression of IL-8, GRO, and MCP-1 which promote vascularization, it exhibits little expression of TNF-α, IL-1β, and INF-γ. Thus, the mature adipocyte-containing composition obtained by the above method is less likely to induce inflammation.

Further, the second aspect of the invention, which includes the mature adipocyte-containing composition as described above, can be used as a substitute for subcutaneous adipose tissues and in augmentation mammaplasty, breast reconstruction surgery, or tissue recess reconstruction surgery. Further, the mature adipocyte-containing composition, which exhibits high expression of IL-8, GRO, and MCP-1 that promote vascularization, can be used as the revascularization promoting agent.

### Brief Description of Drawings

Fig. 1 is a photograph in place of a drawing, showing a culture flask in which microfats crushed by shredding filtration are included.
Fig. 2 is a photograph taken by a phase contrast microscope in place of a drawing, showing mature adipocytes in culture.
Fig. 3 is a photograph in place of a drawing, showing the mature adipocytes in culture subjected to Sudan III staining.
Fig. 4 is a graph in place of a drawing, showing a result of chemokine/cytokine analysis of a conditioned medium.
Fig. 5 is a photograph in place of a drawing, showing a tissue specimen at 6 months after grafting incised from the abdomen of a nude mouse where human suctioned fat pieces alone were grafted.
Fig. 6 is a photograph in place of a drawing, showing a specimen at 6 months after grafting incised from the abdomen of the nude mouse where human adipose tissues, cultured adipocytes, and the conditioned medium were co-grafted.
Fig. 7 is a photograph in place of a drawing, showing a specimen at 6 months after grafting incised from the abdomen of the nude mouse where the human adipose tissue, the cultured adipocytes, and the conditioned medium were co-grafted.
Fig. 8 is a photograph in place of a drawing, showing an example of breast reconstruction surgery after left breast cancer surgery.
Fig. 9 is a photograph in place of a drawing, showing a result of immunostaining for VEGFR2 and PDGFRβ on cultured adipocyte membranes.
Fig. 10 is a graph in place of a drawing, showing the passage number and PDL of the adipocytes.
Fig. 11 is a photograph in place of a drawing, showing the cultured adipocytes on Day 4 of P2 culture grown in α-MEM only (control).
Fig. 12 is a photograph in place of a drawing, showing the cultured adipocytes on Day 4 of the P2 culture grown in a serum-free mesenchymal stem cell growth medium.
Fig. 13 is a photograph in place of a drawing, showing the cultured adipocytes grown in the α-MEM medium supplemented with 10% fetal calf serum (FCS).

### Description of Embodiments

Hereinafter, embodiments of the present invention will be described. The present invention is not limited to the embodiments described below and includes modifications appropriately made based on the following embodiments within a scope obvious to those skilled in the art.

A first aspect of the invention described in this specification is related to a method for producing a mature adipocyte-containing composition. The mature adipocyte-containing composition is, for example, a composition including a human-derived mature adipocyte. For example, Japanese Patent No. 5991687 describes a method for isolating a mature adipocyte. Such a mature adipocyte is publicly known. The size of the mature adipocyte (a length of the part giving the maximum length) may be from 60 to 100 µm, preferably from 1 µm or more to 60 µm or less. The size may be from 5 µm or more to 40 µm or less.

This method includes a shredding-filtration step, a microfat culture step, and a mature adipocyte-containing composition obtaining step.

### Shredding-Filtration Step

The shredding-filtration step is a step of obtaining microfats by shredding and filtering adipose tissues not treated with an enzyme. In a preferable example, the adipose tissues are obtained by washing suctioned fats and then removing blood components. The adipose tissues from which the blood components are removed may be disinfected by using antibiotics, and then the cells may be finely crushed by filtration. The shredding-filtration step preferably includes a step of removing a precipitated stromal vascular fraction (SVF) by performing centrifugal separation. In this case, micro cells are obtained by recovering only fat components floating in an upper part of the liquid phase caused by centrifugal separation. As for centrifugal separation, a publicly known method is appropriately employed.

### Microfat Culture Step

The microfat culture step is a step of obtaining mature adipocytes by culturing the microfats using a culture medium after the shredding-filtration step. These mature adipocytes are included in a cultured cell fraction. The culture medium is a medium containing hydrocortisone and FGF-2. The preferable culture medium is a medium containing self-serum or a medium containing FBS, hydrocortisone, and FGF-2. The culture medium may be a medium containing self-serum, hydrocortisone, and FGF-2.

The medium of the present invention is a basal medium appropriately supplemented with necessary elements. Examples of the basal medium include α-MEM medium, Basal Medium Eagle, and DMEM. The medium is appropriately supplemented with a publicly known reagent used in a medium. Examples of the reagent include fetal bovine serum (FBS), HC (hydrocortisone), FGF2, IGF (insulin-like growth factor), insulin, PDGF (platelet derived growth factor), ACTH (adrenocorticotropic hormone), LIF (leukemia inhibitory factor), TGF β, BMP, a steroid, a fatty acid, soybean trypsin inhibitor, ascorbic acid, hyaluronic acid, proline, dexamethasone, insulin, transferrin, and selenous acid. In a case where ascorbic acid or the like is added to the medium, a salt thereof such as a diphosphate may be added. The reagent is added to each medium in an amount of 0.1 ng/mL or more and 20 µg/mL or less (or 0.2 ng/mL or more and 10 µg/mL or less). These reagents are added by appropriately adjusting their amounts in accordance with a purification degree and a required amount. The self-serum may be added instead of FBS. Further, a serum-free medium may be used. In any case, the medium preferably contains HC (hydrocortisone) and FGF-2.

As an example of the culture medium, α-MEM medium is supplemented with 1 to 10% fetal bovine serum (FBS), from 20 ng/ml to 100 ng/ml, both inclusive, of hydrocortisone, and from 5 ng/ml to 20 ng/ml (or 50 ng/ml), both inclusive, of FGF2 (fibroblast growth factor 2). 1 to 10% self-serum may be added together with or instead of 1 to 10% fetal bovine serum (FBS). These amounts may be appropriately adjusted. For example, FBS and self-serum may be included in the medium in an amount of from 0.1% to 20%.

The microfats may be cultured and grown under normal culture conditions. As a cell amount, the culture is allowed to reach about 10% to 100% confluent state. Further, the cells can be cultured at high density in a multilayer state to over 100% confluency. The cells may be transferred to low oxygen conditions immediately after grafting or after being left still for a while. The low oxygen culture can be performed, for example, by using a low oxygen incubator in which commercially available nitrogen gas or the like is mixed to reduce an oxygen partial pressure or by blowing nitrogen gas or the like into an appropriate space to reduce an oxygen partial pressure.

The culture is preferably performed under conditions of 3% or more and 20% or less carbon dioxide gas and 1% or more and 10% or less oxygen gas. The culture may be performed under conditions of 5% or more and 20% or less carbon dioxide gas and 2% or more and 10% or less oxygen gas, or 8% or more and 12% or less carbon dioxide gas and 3% or more and 7% or less oxygen gas.

### Mature Adipocyte-Containing Composition Obtaining Step

The mature adipocyte-containing composition obtaining step is a step of obtaining the mature adipocyte-containing composition including the mature adipocytes and the conditioned medium obtained through the microfat culture step. The mature adipocyte-containing composition may include the mature adipocytes and the culture supernatant.

In a preferable example, the mature adipocytes express each of IL-8, GRO, and MCP-1, a family of chemokines/cytokines, 4 times or more higher than IL-6. The mature adipocytes may express any of IL-8, GRO, and MCP-1 or two or more kinds thereof 10 times or more higher than IL-6, 20 times or more higher than IL-6, or 20 times or more higher than IL-6. The mature adipocyte-containing composition, which includes such chemokines/cytokines having high revascularization promoting ability and low inflammatory induction properties, is suitable for grafting as it exhibits high vascularization promoting ability without inducing inflammation when grafted. It is particularly preferable that this mature adipocyte-containing composition includes fats collected from a patient to whom grafting is performed.

In a preferable example, the mature adipocytes express any of IL-8, GRO, and MCP-1 or two or more kinds thereof 10 times or more higher than TNF-α, preferably 50 times or more higher than TNF-α, preferably 100 times or more higher than TNF-α, preferably 500 times or more higher than TNF-α.

In a preferable example, the mature adipocytes express any of IL-8, GRO, and MCP-1 or two or more kinds thereof 10 times or more higher than IL-1-β, preferably 50 times or more higher than IL-1-β, preferably 100 times or more higher than IL-1-β, preferably 500 times or more higher than IL-1-β.

In a preferable example, the mature adipocytes express any of IL-8, GRO, and MCP-1 or two or more kinds thereof 10 times or more higher than INF-γ, preferably 50 times or more higher than INF-γ, preferably 100 times or more higher than INF-γ, preferably 500 times or more higher than INF-γ.

The mature adipocyte-containing composition includes appropriate amounts of the mature adipocytes and the conditioned medium (or the culture supernatant) depending on purposes. Further, this composition, like a normal composition, is stored in a required container and used as needed. The mature adipocytes may be used, for example, in an amount of from 1 × 10⁴ cells to 1 × 10¹⁰ cells, both inclusive, per single application, or from 1 × 10⁵ cells to 1 × 10⁸ cells, both inclusive, per single application.

An agent including the culture supernatant as an active ingredient is publicly known as disclosed in, for example, JP 2013-18756 A, Japanese Patent No. 5139294, and Japanese Patent No. 5526320. Thus, the composition including the conditioned medium can be produced by using the publicly known methods.

Examples of the culture supernatant of the mature adipocytes include a treated material obtained by removing the water content by freeze-drying from the culture supernatant, which is a supernatant component obtained by subjecting the culture supernatant to solid-liquid separation by centrifugal separation, a treated material obtained by concentrating the culture supernatant under reduced pressure by using an evaporator or the like, a treated material obtained by concentrating the culture supernatant by using a ultrafiltration membrane or the like, a treated material obtained by subjecting the culture supernatant to solid-liquid separation by using a filter, or an undiluted solution of the culture supernatant not treated by the above treatments. Further, for example, the sterile culture supernatant may be obtained by subjecting a supernatant obtained by culturing the mature adipocytes of the present invention to centrifugal separation (e.g., 1,000 x g, 10 min.), performing fractionation using ammonium sulfate (e.g., 65% saturated ammonium sulphate), suspending the resulting precipitate in an appropriate buffer solution, subjecting the resulting suspension to a dialysis treatment, and filtering the resulting suspension with a syringe filter (e.g., 0.2 µm). The collected culture supernatant can be used as it is or frozen and stored to be used later by thawing. Further, a pharmaceutically acceptable carrier may be added to the culture supernatant and the culture supernatant may be aliquoted into sterile containers in a liquid amount to be easily handled. Further, the culture supernatant may be treated with a virus clearance filter or ultraviolet irradiation as measures against an infectious pathogen risk. The mature adipocyte-containing composition includes the culture supernatant in an amount of preferably 1 mL or more and 1,000 mL or less, more preferably 30 mL or more and 300 mL or less, as a single administration unit.

In a preferable example, the mature adipocyte-containing composition is used as a substitute for subcutaneous adipose tissues. This specification also provides a method for substituting a subcutaneous adipose tissue including a step of administering the mature adipocyte-containing composition to a human patient. It is preferable that the mature adipocyte-containing composition in this method further includes adipose tissues derived from the patient to whom the method is applied in addition to the mature adipocytes and the conditioned medium. Further, the mature adipocyte-containing composition may be used in augmentation mammaplasty, breast reconstruction surgery, or tissue recess reconstruction surgery. For example, breast reconstruction surgery includes a step of injecting the mature adipocyte-containing composition into the breast deficient in fat components.

A second aspect of the invention described in this specification is related to a composition used as a substitute for subcutaneous adipose tissues. This composition is the mature adipocyte-containing composition produced by any of the above methods for producing the mature adipocyte-containing composition and used as a substitute for subcutaneous adipose tissues. This composition is used in, for example, augmentation mammaplasty, breast reconstruction surgery, or tissue recess reconstruction surgery.

A third aspect of the invention described in this specification is related to a revascularization promoting agent. This revascularization promoting agent includes the mature adipocyte-containing composition obtained by, for example used as, any of the above methods for producing the mature adipocyte-containing composition. That is, this revascularization promoting agent is the same as the mature adipocyte-containing composition described above. The revascularization promoting agent may be, for example, an injection. The mature adipocyte-containing composition described above is loaded in an injection syringe, optionally mixed with tissues derived from the patient, and grafted to a required site of the patient.

### Example 1

### Culture Method: Cultured Fat Medium Composition

After obtaining informed consent, adipose tissues were suctioned and collected from the abdomen, the thigh, the hip, and the upper arm using a cannula with a dimeter of 2 mm or less (the diameter is not limited). The suctioned and collected fats were washed and their blood components were removed. These tissues were disinfected using antibiotics and then subjected to shredding filtration to finely crush the fats. After centrifugation, only fat components floating in an upper part of the liquid phase were cultured. After centrifugation, the SVF precipitated in a bottom layer as a pellet was removed.

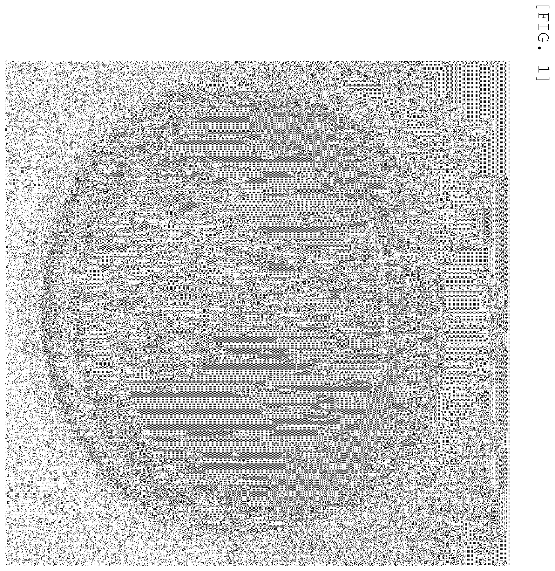

These microfats were placed in a culture flask to start culture. Fig. 1 is a photograph in place of a drawing, showing the culture flask in which the microfats crushed by shredding filtration are placed. As shown in Fig. 1, only the fat components remain after the SVF is removed. The amount of the medium in culture was adjusted to a small quantity to prevent the tissues from floating. After confirming cell adhesion in some cells from the tissue pieces on Day 5 of culturing, more medium was added to continue culturing.

The medium in use was α-MEM medium supplemented with 5% fetal bovine serum (FBS), 40 ng/ml hydrocortisone, and 10 ng/ml FGF2 (Fibroblast Growth Factor 2). The culture was performed under conditions of 10% carbon dioxide gas and 5% oxygen gas.

On Day 12 of the primary culture, 2 to 3 × 10⁶ cultured cells were recovered. A fraction of these cells was frozen and the remaining cells were inoculated at a density of 4 to 5 × 10⁴/cm² and cultured for 5 days. After 6 days, the cells were washed 3 times with PBS (-) and cultured for 2 days in a medium in which FBS was removed from the above medium components. This medium was used for analysis as a conditioned medium.

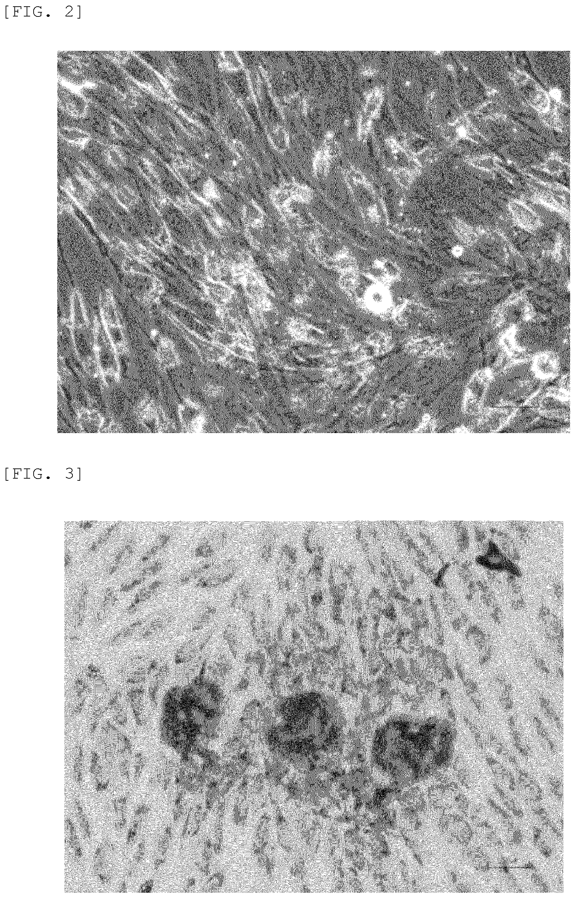

Fig. 2 is a photograph taken by a phase contrast microscope in place of a drawing, showing the mature adipocytes in culture. As shown in Fig. 2, the adipocytes contained a large quantity of fat particles. Sudan III staining was performed to confirm the mature adipocytes in culture. The mature adipocytes are specifically stained red by Sudan III which is used to specifically stain fats. The result is shown in Fig. 3. Fig. 3 is a photograph in place of a drawing, showing the mature adipocytes in culture stained by Sudan III. As shown in Fig. 3, the medium contains a large quantity of the fat particles.

### Example 2

The conditioned medium of the cultured mature adipocytes was subjected to chemokine/cytokine analysis by a method using antibody-immobilized magnetic beads. A supernatant was obtained from a conditioned medium sample by centrifugation at 13,000 G at 4°C for 5 minutes and used for measurement. Concentration of 40 target proteins in the conditioned medium was measured using the Luminex (registered trademark) System. Each pretreated sample in 25 µL was applied to a single well, and the measurement was performed 3 times. One standard solution was included in accordance with the manual, and 5-fold serial dilutions were prepared for a total of 7 points and measured 3 times. Forty target proteins were as follows: EGF, FGF-2, Eotaxin, TGF-α, G-CSF, Flt-3L, GM-CSF, fractalkine, IFNα2, IFNγ, GRO, IL-10, MCP-3, IL-12p40, MDC, IL-12p70, PDGF-AA, IL-13, PDGF-AB/BB, IL-15, sCD40L, IL-17A, IL-1RA, IL-1α, IL-9, IL-1β, IL-2, IL-3, IL-4, IL-5, IL-6, IL-7, IL-8, IP-10, MCP-1, MIP-1α, MIP-1β, RANTES, TNFα, TNFβ, and VEGF.

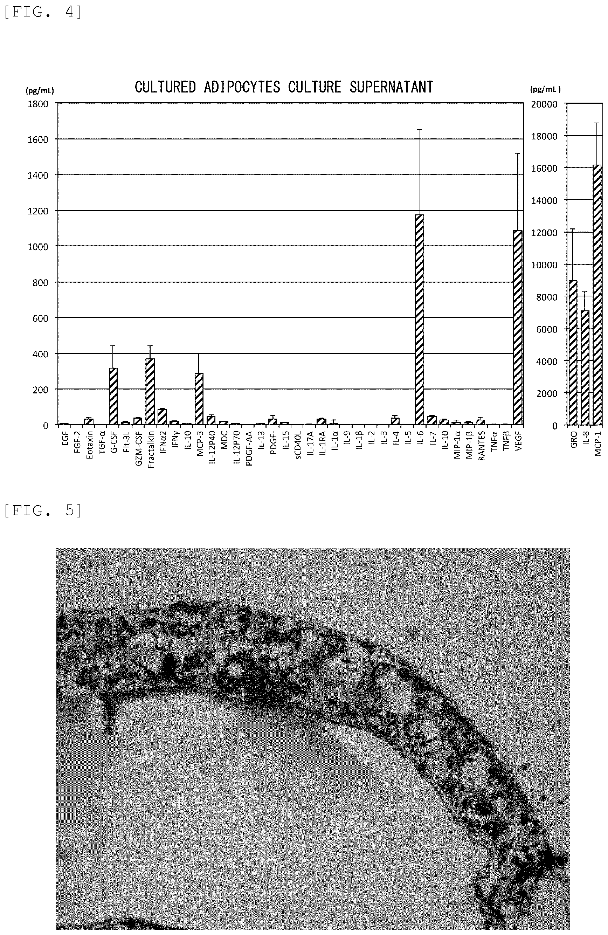

Fig. 4 is a graph in place of a drawing, showing a result of the chemokine/cytokine analysis of the conditioned medium. As shown in Fig. 4, it is proved that the conditioned medium of the cultured mature adipocytes produces a large quantity (ng/ml) of IL-8, GRO, and MCP-1, which are cytokines having high revascularization ability. On the other hand, TNF-α, which is increased in adipose-derived stem cells and causes inflammation, and IL-1-β and INF-γ having strong inflammatory induction properties are hardly produced in these cells, proving their safety.

### Example 3

### Fat Grafting Tests into Nude Mice

### Mouse Grafting Tests and Evaluation of Grafted Tissue Images

After obtaining informed consent, adipose tissues were suctioned and collected from the abdomen. The tissues were disinfected and inoculated by the same method as described above. On Day 12 of the primary culture, 2 × 10⁶ cultured adipocytes were recovered. These cells were then frozen and stored. Prior to grafting, the cells were thawed, inoculated at a density of 5 × 10⁴ cells/cm², and cultured for 5 days. The 3 × 10⁶ cultured adipocytes recovered by an enzyme treatment were added and mixed with 0.6 to 0.7 g of fats to be grafted, and the mixture was grafted subcutaneously in the backs of nude mice next day. The fats alone were grafted in mice in a control group. The tissues were removed 6 months after grafting and subjected to tissue staining. While most adipose tissues were absorbed when the human adipose tissues alone were grafted, the adipose tissues perfectly survived when the cultured cells and the human fats were co-grafted. Furthermore, formation of the blood vessels was observed in the central parts.

Tissue specimens at 6 months after grafting, incised from the abdomens of the nude mice where the human suctioned fat pieces alone were grafted, were stained using Oil Red O. Fig. 5 is a photograph in place of a drawing, showing the tissue specimen at 6 months after grafting incised from the abdomen of the nude mouse where the human suctioned fat pieces alone were grafted. Fig. 5 shows the image taken at 100 times magnification. Although adipose tissues are formed in some parts of the periphery, hollows in the central parts are sites where the fats are absorbed after grafting. Around the hollows, scar tissues and some of the fats cause formation of tissues.

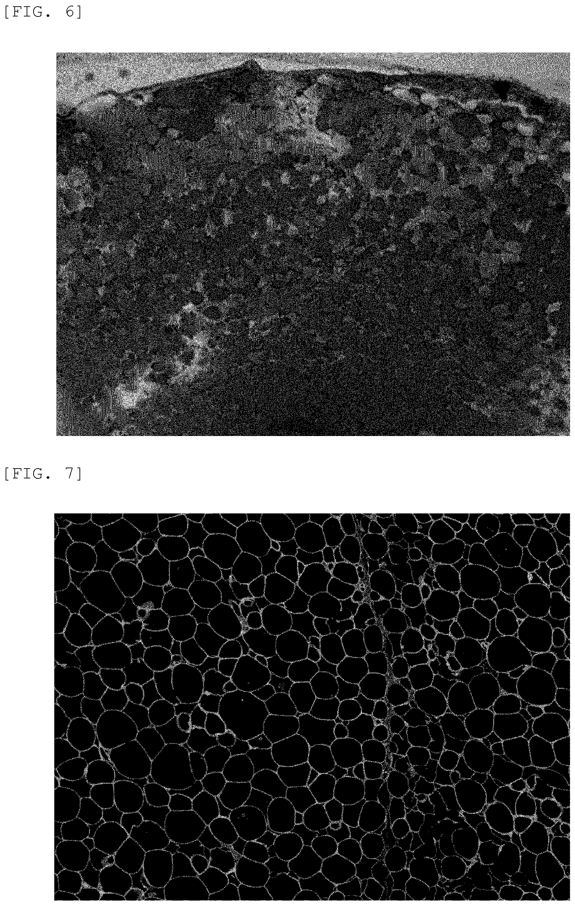

Specimens at 6 months after grafting, incised from the abdomens of the nude mice where the human adipose tissues, the cultured adipocytes, and the conditioned medium were co-grafted, were stained using Oil Red O. Fig. 6 is a photograph in place of a drawing, showing the specimen at 6 months after grafting incised from the abdomen of the nude mouse where the human adipose tissues, the cultured adipocytes, and the conditioned medium were co-grafted. Fig. 6 shows the image taken at 100 times magnification. The adipose tissues are formed in 95% or more of the whole specimen tissue. Vascular tissues were observed inside the adipose tissues.

The human adipose tissues, the cultured adipocytes, and the conditioned media were co-grafted to the abdomens of the nude mice. Staining was performed using immunofluorescence staining (Human Nuclear Antigen Antibody 231-1 [Alexa Fluor (registered trademark) 488], Novus Biologicals, Southpark Way, USA) to confirm that the fats were human-derived cells. Fig. 7 is a photograph in place of a drawing, showing the specimen at 6 months after grafting incised from the abdomen of the nude mouse where the human adipose tissues, the cultured adipocytes, and the conditioned medium were co-grafted. The adipose tissues are formed in the entire specimen tissue. Blood vessel-like tissues were also observed inside the adipose tissues.

### Example 4

### Fat Grafting to Human (Mastectomy/Breast Reconstruction Surgery)

### Example 4-1: Grafting Example of Cultured Mature Adipocytes, Conditioned Medium, and Self-Fats for Breast Reconstruction After Left Breast Cancer Surgery

For 46-year-old female who had left breast cancer surgery, a tissue expander was placed in the chest during excision of the breast cancer for the expansion of the subcutaneous tissues and the pectoralis major, thereby increasing the size of the breast. Subsequently, as per the patient's request, the present method was scheduled. From the abdomen, 2 cc of fats were suctioned and collected, disinfected, and inoculated for culturing. On Day 14 of the primary culture, 3 × 10⁶ cultured adipocytes were recovered. These cultured adipocytes were then frozen and stored. Prior to grating, the cells were thawed and cultured for 6 days in 12 flasks each having a bottom area of 150 cm². The suctioned adipose tissues, the cultured mature cells, and the conditioned medium to be used for grafting were mixed together and grafted. In the first grafting, a total of 252 ml (17.16 × 10⁷ cells) was directly, subcutaneously grafted into the left chest at the periphery of the subcutaneous tissue expander. About 6 months later, when the tissue expander was removed, a total of 258 ml (11.44 × 10⁷ cells) corresponding to 8 flasks was directly, subcutaneously grafted into the left chest in an inner cavity and at the periphery of the inner cavity.

As a medium for the primary culture and culturing the thawed cells, a medium supplemented with 10% self-serum, 40 ng/ml hydrocortisone, and 210 ng/ml FGF was used. Note that it was confirmed that the same result was obtained by using FBS instead of 10% self-serum.

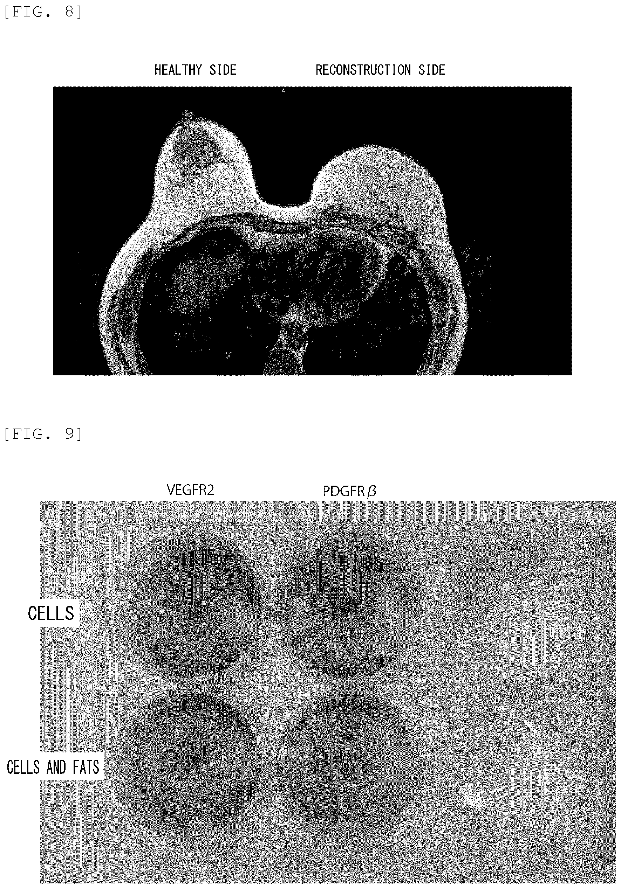

An image analysis of the grafted adipose tissues was performed by MRI one year after grafting. As a result, it was shown that the grafted adipose tissues remained the original size without being absorbed. The result is shown in Fig. 8. Fig. 8 is a photograph in place of a drawing, showing an example of the breast reconstruction surgery after the left breast cancer surgery. The MRI image is a horizontal cross section taken from a lower side and shows the mammary gland tissue and a nipple on the healthy side. On the reconstruction side, nearly 100% of the grafted fats survive without being absorbed. White brightness in the adipose tissues indicates that the fats are formed throughout the whole layer. Further, it is observed that many blood vessels travel inside the adipose tissues. There is no fat necrosis, cyst, calcification, or stiffness (induration) observed. The fat formation was reliably observed (the same results were obtained in other 10 cases). The image was taken in a face-down position, thus the breast on the healthy side is hanging down.

### Fat Grafting to Human (Breast Conserving Reconstruction Surgery)

### Example 4-2: Grafting Example of Culture Mature Adipocytes, Conditioned Media, and Self-Fats After Left Breast Cancer Conserving Surgery and for Right Breast Atrophy and Hypoplasia

A 44-year-old female who had the left breast cancer surgery had diagnosis of severe breast recess deformation (deformation by half or more) after the left breast cancer conserving surgery, and the right breast hypoplasia and atrophy. As per the patient's request, the present method was scheduled. From the abdomen, 2 cc of fats were suctioned and collected, disinfected, and inoculated for culturing. On Day 14 of the primary culture, 3.4 × 10⁶ cultured cells were recovered. These cultured cells were then frozen and stored. Prior to grating, the cells were thawed and cultured for 7 days in 15 flasks each having a bottom area of 150 cm². As a medium for the primary culture and culturing the thawed cells, a medium supplemented with 10% self-serum, 40 ng/ml hydrocortisone, and 210 ng/ml FGF was used. The suctioned adipose tissues, the cultured adipocytes, the cultured mature cells, and the conditioned medium to be used for grafting were mixed together and grafted. The total graft amount was 310 ml (21.45 × 10⁷ cells). A total of 220 ml was injected and grafted throughout the subcutaneous tissues of the left breast cancer conserving part. The remaining 90 ml was injected and grafted in the right breast atrophy and hypoplasia part. One year after grafting, the adipose tissues remained the original size without being absorbed. Stiffness (induration) or the like was not observed, and the external appearance was excellent.

### Fat Grafting to Human (Facial Recess Deformation Reconstruction Surgery)

### Example 4-3: Grafting Example of Cultured Mature Adipocytes, Conditioned Media, and Self-Fats for Facial Atrophy

A 68-year-old female who had the left breast cancer surgery had diagnosis of recess deformation caused by the right facial atrophy. Further, the fats had been suctioned from the abdomen, the thigh, and the like in other clinics, making it difficult for this case to perform normal fat collection. As per the patient's request, the present method was scheduled. From the abdomen, 2 cc of fats were suctioned and collected, disinfected, and inoculated for culturing. On Day 16 of the primary culture, 4.4 × 10⁶ cultured cells were recovered. These cultured cells were then frozen and stored. Prior to grating, the cells were thawed and cultured for 7 days in 5 flasks each having a bottom area of 175 cm². As a medium for the primary culture and culturing the thawed cells, a medium supplemented with 10% self-serum, 40 ng/ml hydrocortisone, and 210 ng/ml FGF was used. The suctioned adipose tissues, the cultured cells, the cultured mature cells, and the conditioned medium to be used for grafting were mixed together and grafted. The total graft amount was 35 ml (8.35 × 10⁷ cells). One year after grafting, the adipose tissues remained the original size without being absorbed. Stiffness (induration) or the like was not observed, and the external appearance was excellent.

Further, pectus excavatum is a condition in which the chest wall recess deformation occurs in the chest and can be considered as medically severe recess deformation, like the case in Example 4-2. Thus, pectus excavatum is included in indications for the use of the present method.

### Discussions

According to the conventional concept, it is thought that the SVF (ASCs) including the adipose-derived stem cells promotes neovascularization, thus grafting the SVF (ASCs) with the fats increases the graft survival rate. In animal studies, the viable zone of the graft pieces is about 1.5 ±0.5 mm from the edge and a loss of 60% of the adipocytes was observed among the viable cells. Even if the adipose derived stromal cells (ASCs) included in the SVF were added to the adipocytes, the survival zone of only less than 300 µm could be obtained. Further, in human studies, it has been reported that the breast fat grafting, a procedure termed cell-assisted lipotransfer (CAL), is not different from the conventional fat grafting in terms of the survival rate, or, controversially, it has been reported that the survival rate was 39% for the conventional fat grafting and 63% for the CAL (Toyserkani MN., Quaade ML, and Sorensen JA. Cell-Assisted Lipotransfer: A Systematic Review of Its Efficacy. Aesthetic Plast Surg. 2016; 40: 309-318). The survival rate of the CAL applied to the breast was described in 5 reports among 10 reports and varies as follows: 51.8%, 47%, 40% to 80%, 54%, and 50% (Cell assisted lipotransfer in breast augmentation and reconstruction: A systematic review of safety, efficacy, use of patient reported outcomes and study quality. Arshad Z, Karmen L, Choudhary R, Smith JA, Branford OA, Brindley DA, Pettitt D, and Davies BM. JPRAS OPEN. 2016 Dec; 10: 5-20).

Vascularization ability of GRO is described in the literatures (Caunt M,1 Liang Hu,1 Thomas Tang,1 Peter C. Brooks,2 Sherif Ibrahim,3 and Simon Karpatkin. Growthregulated Oncogene Is Pivotal in Thrombin-Induced Angiogenesis. Cancer Res 2006; 66(8): 4125-32, Keglowich1 L, Roth M, Philippova1 M, Resink T, Tjin G, Bronchial Smooth Muscle Cells of Asthmatics Promote Angiogenesis Through Elevated Secretion of CXC-chemokines (ENA-78, GRO-α, and IL-8) PLoS One. 2013; 8(12): e81494. doi: 10.1371/journal.pone.0081494).

Vascularization ability of IL-8 is also described in the literatures (Mikula-Pietrasik J, Kuczmarska A, Kucińska M, Muriaset M. et al. Resveratrol and its synthetic derivatives exert opposite effects on mesothelial cell-dependent angiogenesis via modulating secretion of VEGF and IL-8/CXCL8. Angiogenesis 2012; 15: 361-376 and Keglowich1 et al. described above).

MCP-1 is reported to be a chemokine for neovascularization, and vascularization ability of MCP-1 is described in another literature (6. Niu J, Azfer A, Zhelyabovska O, Fatma S, and Kolattukudy PE. Monocyte Chemotactic Protein (MCP)-1 Promotes Angiogenesis via a Novel Transcription Factor, MCP-1-induced Protein (MCPIP). J Biol Chem 2008 May 23; 283(21): 14542-51. doi: 10.1074/jbc.M802139200). Further, it is described that MCP-1 mediates vascularization ability of VEGF in the literature (3. Hong KH, Ryu J, Han KH. Monocyte Chemoattractant protein-1-induced Angiogenesis Is Mediated by Vascular Endothelial Growth factor-A. Blood 2005; 105: 1405-1407 DOI:10,1182/blood-2004-08-3178). Vascularization requires mobilization of macrophages, and MCP-1 is an essential factor for performing this process.

Collectively, it is shown that all 4 kinds of cytokines (MCP-1, IL-8, GRO, and VEGF) included in the conditioned medium, secreted by the cultured adipocytes in Examples, have vascularization ability.

### Example 5

The cultured mature adipocytes P2 were inoculated at 1 × 10⁴/6-well (8 cm²/well) and then a group of the cultured mature adipocytes alone and a co-culture group consisting of the cultured mature adipocytes and the suctioned fats were created and cultured for 5 days, followed by immunostaining for VEGFR2 (vascular endothelial growth factor receptor 2) and PDGFRβ (platelet-derived growth factor beta). Reagents in use were anti-VEGFR2 polyclonal antibody (Funakoshi Co., Ltd.: bs-10412), anti-PDGFR beta polyclonal antibody [Y92]-C-terminal (ab32570), goat anti-rabbit IgG H&L (HRP) (ab205718), and DAB substrate kit (Funakoshi Co. Ltd.: CSK-4100, VEC).

The obtained result is shown in Fig. 9. It is shown that both groups contain cells strongly expressing VEGFR2 and PDGFRβ.

Since VEGFR2 is a primary responder of the VEGF signal and expressed only in vascular endothelial cells and their progenitor cells, it is shown that the blood vessels are formed. PDGFRβ is expressed in the adipose progenitor cells when PPAPγ is stimulated by a fatty acid or the like. Thus, the obtained result shows that the adipose progenitor cells are also contained. It is thought including these two cells simultaneously promotes vascularization and fat formation after grafting. Further, considering that the aforementioned vascularization promoting factors (MCP-1, IL-8, GRO, and VEGF) are abundantly secreted, it is found that vascularization after grafting is driven by two wheels, i.e., cell types and factors, thereby exhibiting very strong neovascularization activity. Further, it is shown that VEGFR2 and PDGFRβ are strongly expressed also in the co-culture group consisting of the mature adipocytes and the suctioned fats.

### Example 6

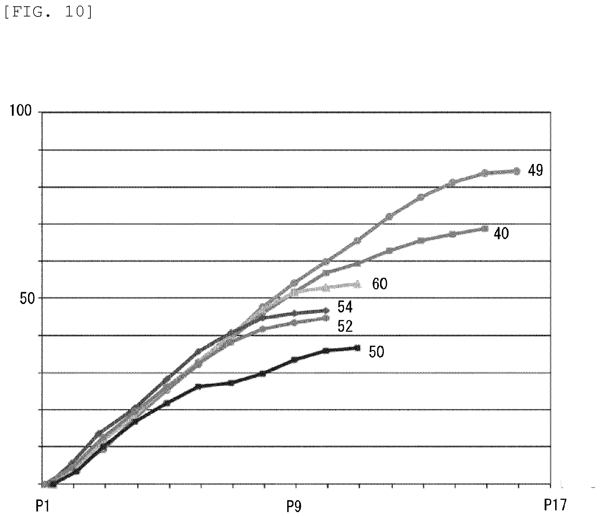

Tumorigenic cells are characterized by keep growing without being arrested. In order to confirm the safety of the cultured adipocytes generated in Examples, aging of the adipocytes was tested. This test is for determining when the adipocytes stop growing after being continuously cultured and passaged.
The cultured adipocytes derived from 6 subjects were cultured for 3 months and PDL (CPD) until growth arrest was measured. These indexes indicate how many times cells divide. The result is shown in Fig. 10. Fig. 10 is a graph in place of a drawing, showing the passage number and PDL of the adipocytes. As shown in Fig. 10, it is confirmed that the growth arrest is caused by aging. P14 corresponding to a total of 102-day culture: vertical axis indicating CDL (or PDL)
49-year-old (arrest on Day 102), 40-year-old (arrest on Day 95), 60-year-old (arrest on Day 67), 54-year-old (arrest on Day 61), 52-year-old (arrest on Day 62), 50-year-old (arrest on Day 67)

### Example 7

An experiment was performed to test if the cultured adipocytes can be cultured in a serum-free medium. The cultured adipocytes could grow in a commercially available serum-free mesenchymal stem cell growth medium (Lonza PT-3001: Lonza. KK) although less proliferative than the medium with fetal calf serum.

### Basal Media:

A: α-MEM only, serum free (control)
   Concentration of HC and FGF to be added is the same as in clinical conditions.
B: Medium supplemented with serum-free mesenchymal stem cell growth medium (Lonza PT-3001)
C: α-MEM supplemented with 10% fetal calf serum (FCS) (positive control)

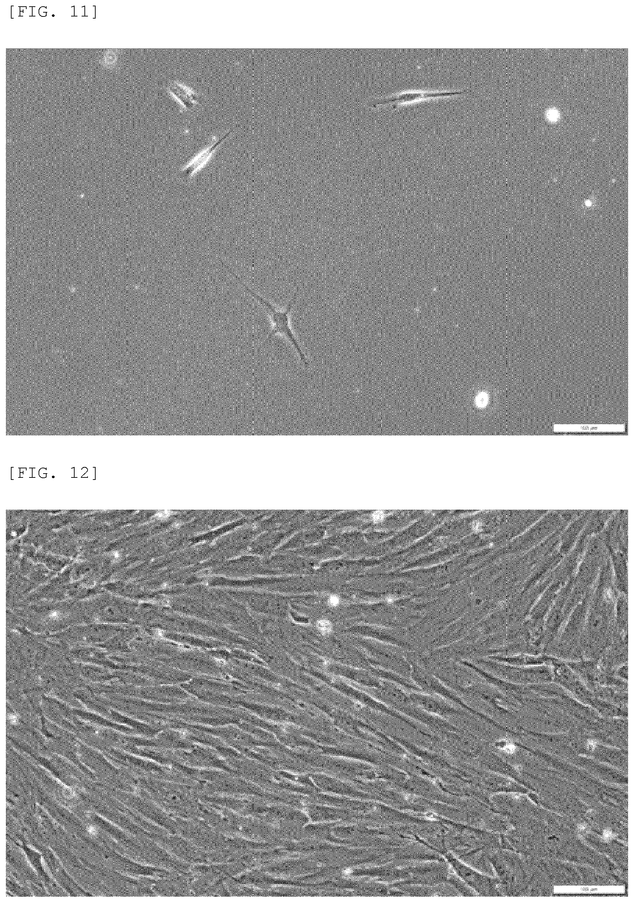

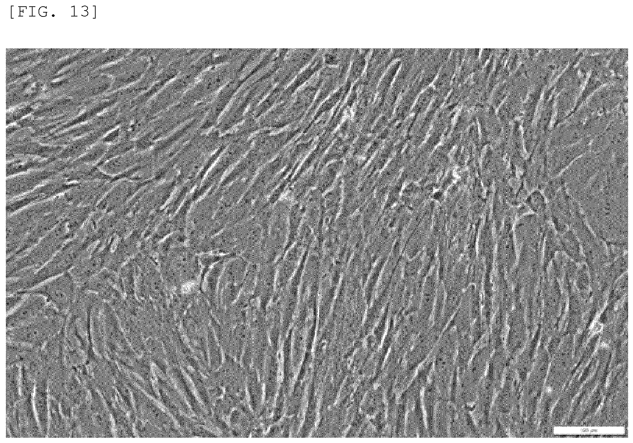

The above media were compared and examined. The result is shown in Fig. 11 to Fig. 13. Fig. 11 is a photograph in place of a drawing, showing the cultured adipocytes on Day 4 of P2 culture grown in α-MEM only (control). Fig. 12 is a photograph in place of a drawing, showing the cultured adipocytes on Day 4 of P3 culture grown in the serum-free medium (Lonza PT-3001). Fig. 13 is a photograph in place of a drawing, showing the cultured adipocytes grown in the α-MEM supplemented with 10% fetal calf serum (FCS). Table 1 shows the number of cells in each medium.

**[Table 1]**

| Cells counted on Day 4 after P3 inoculation | | | | | |
|---|---|---|---|---|---|
| | Number of inoculated cells | Number of cells | Number of SaverageSD | PDL | PDL average SD |
| α-MEM medium | | | | | |
| 1 | 10000 | 1300 | 1533.33±251.66 | -2,94 | -2,72±0.24 |
| 2 | 10000 | 1800 | | -2,47 | |
| 3 | 10000 | 1500 | | -2.74 | |

| Lonza Serum-free medium 40ng/mL HC, 10ng/mL FGF | | | | | |
|---|---|---|---|---|---|
| 1 | 10000 | 77000 | 78666.67 ± 3785,94 | 2,94 | 2,97±0.07 |
| 2 | 10000 | 83000 | | 3,05 | |
| 3 | 10000 | 76000 | | 2.93 | |

| FCS 10%+*α*-MEMmedium+40ng/mL. HC, 10ng/mL FGF | | | | | |
|---|---|---|---|---|---|
| 1 | 10000 | 110000 | 120000.00±10000.00 | 3,46 | 3.58±0.12 |
| 2 | 10000 | 130000 | | 3,70 | |
| 3 | 10000 | 120000 | | 3.58 | |

| | | | | | |
|---|---|---|---|---|---|
| ∗ PDL formula = LOG(yield number/inoculation number)/LOG(2) | | | | | |

### Industrial Applicability

This invention can be used in the field of medical equipment and the like.

## Claims

1. A method for producing a mature adipocyte-containing composition comprising:
a shredding-filtration step for obtaining a microfat by shredding and filtering an adipose tissue without treating with an enzyme;
a microfat culture step for obtaining a mature adipocyte by culturing the microfat using a culture medium after the shredding-filtration step; and
a mature adipocyte-containing composition obtaining step for obtaining a mature adipocyte-containing composition including the mature adipocyte and a conditioned medium obtained through the microfat culture step, wherein:
the shredding-filtration step includes a step of removing a precipitated stromal vascular fraction (SVF) by performing centrifugal separation; and
the culture medium is a medium containing hydrocortisone and FGF-2.

2. The method for producing the mature adipocyte-containing composition according to claim 1, wherein the adipose tissue is obtained by washing a suctioned fat and then removing a blood component.

3. The method for producing the mature adipocyte-containing composition according to claim 2, wherein the shredding-filtration step further includes a step of obtaining the micro cell by recovering a fat component floating in an upper part of a liquid phase caused by the centrifugal separation.

4. The method for producing the mature adipocyte-containing composition according to claim 3, wherein the conditioned medium includes each of IL-8, GRO, and MCP-1 4 times or more higher than IL-6.

5. The method for producing the mature adipocyte-containing composition according to claim 3, wherein the conditioned medium includes vascular endothelial growth factor (VEGF) 0.5 times or more to 1.5 times or less higher than IL-6.

6. The method for producing the mature adipocyte-containing composition according to any of claims 1 to 5, wherein the mature adipocyte-containing composition is used as a substitute for a subcutaneous adipose tissue.

7. The method for producing the mature adipocyte-containing composition according to any of claims 1 to 5, wherein the mature adipocyte-containing composition is used in augmentation mammaplasty, breast reconstruction surgery, or tissue recess reconstruction surgery.

8. A method for producing a revascularization promoting agent including the mature adipocyte-containing composition obtained by the method for producing the mature adipocyte-containing composition according to any of claims 1 to 5.

9. The method for producing the revascularization promoting agent according to claim 8, wherein the mature adipocyte-containing composition includes vascular endothelial growth factor receptor (VEGFR) 2 or a positive cell thereof and platelet-derived growth factor receptor (PDGFR) β.
